# EUROPEAN PATENT APPLICATION

(11) **EP 3 409 663 A1**
(43) Date of publication of application: **05.12.2018**
(21) Application number: 18386015.4
(22) Date of filing: 31.05.2018
(51) Int. Cl.: C07D 307/92, C07C 35/36

(54) **CONVERSION OF SCLAREOL TO MANOYL OXIDE UNDER MORDENITE CATALYSIS**

(30) Priority: 01.06.2017 GR 20170100252
(71) Applicant: Vioryl Chemical and Agricultural Industry, Research S.A., 19014 Afidnes (GR)
(72) Inventor: MAKAROUNI, Dimitra-Panagiota, 14564 Athens (GR)

(57) **Abstract**

The present invention relates to a method using a novel catalytic system based on the combination of mordenite (drawing 4) with glycol ether, for the preparation of cyclic ethers from tertiary alcohols. This novel catalytic system is applied for the selective one step conversion (drawing 1) of Sclareol (I) (Labd-14-ene-8,13-diol) to Manoyl oxide (II) (8,13-epoxy-labd-14-ene) (drawing 2) in yields higher than 85% (drawing 3). This new catalytic system is based on the combination of a Mordenite catalyst along with a polar, aprotic, high boiling point solvent that belongs to the glyme type ethers (Dimethoxyethane). The solvent of choice can be diglyme(Diethylene glycol dimethyl ether), Proglyme (Dipropylene glycol dimethyl ether), Tetraglyme (Tetraethylene glycol dimethyl ether).

## Description

The present invention relates to a method using a novel catalytic system based on the combination of mordenite with glycol ether, for the preparation of cyclic ethers from tertiary alcohols. This novel catalytic system is applied for the selective one step conversion of Sclareol (I) (Labd-14-ene-8,13-diol) to Manoyl oxide (II) (8,13-epoxy-labd-14-ene) in yields higher than 85%. This new catalytic system is based on the combination of a Mordenite catalyst along with a polar, aprotic, high boiling point solvent that belongs to the glyme type ethers (Dimethoxyethane). The solvent of choice can be Diglyme(Diethylene glycol dimethyl ether), Proglyme (Dipropylene glycol dimethyl ether), Tetraglyme (Tetraethylene glycol dimethyl ether).

In previous studies, the conversion of certain diols to ether derivatives through heterogeneous catalysis has been extensively shown. Some of the methods which claim to produce cycloether derivatives by the cyclodehydration of 1,5-diols, require a high pressure system which is a drawback compared to the method described in the present invention, where atmospheric pressure and temperatures not higher than 140°C are applied. In addition, in other patents which describe the cyclodehydration of diols for the production of cyclic ethers, there is the limitation that at least one of the hydroxyl groups of the diol must be non-tertiary which is also a disadvantage compared to the present invention in which there is not such restriction. Finally, a catalytic system consisting of Mordenite along with a glycol ether for the production of cyclic ethers from tertiary alcohols has not ever been reported in the prior art.

Manoyl oxide (II) which belongs to labdane diterpenes, exhibits a number of important properties. Manoyl oxide is a precursor of the compound Forskolin and other medically important labdane-type terpenoids (Appl Environ Microbiol. 2014 Dec; 80(23): 7258-7265. doi: 10.1128/AEM.02301-14). Since the first reported synthesis of Manoyl oxide, a number of synthetic procedures have been proposed. Many of them starting from Sclareol (I), which is a diterpene found in clary sage (Salvia sclarea), from which it derives its name.

Prior art on the synthesis of Manoyl oxide comprises in patents such as WO2016075302A1 where the biosynthesis of Manoyl oxide or (13R)-Manoyl oxide are reported. In addition, there is published scientific work on the synthesis of Manoyl oxide which however comprises only low yields or multistep treatment (Plant Physiology March 2014 vol. 164 no. 3 1222-1236, doi: http://dx.doi.org/10.1104/pp.113.228429), (Khimiya Prirodnykh Soedinenii, Vol. 2, No. 3, pp. 176-179, 1966 doi: 10.1007/BF01134233), (Synthetic communications, 23(21), 2991-2998 (1993) http://dx.doi.org/10.1080/00397919308011142).

Furthermore, in patents such as US9469622B2 the cyclodehydration of diols over solid catalysts is described in order to produce Ambrafuran (3a,6,6,9a-Tetramethyldodecahydronaphtho-[2,1-b]furan) and other cycloether derivatives. According to some embodiments in the aforementioned patent, the conversion of Sclareol to other terpenes is achieved biosynthetically, where it is converted to Sclareolide (III) and Ambrafuran and not in any case to Manoyl oxide. Manoyl oxide is not identified or included in the MS spectrum of any of the products.

As a result from the above, there is the need for an improved process for the production of Manoyl oxide from Sclareol under the scope of:
Avoiding multistep chemical reactions and developing a one-step reliable process.
Avoiding the use of homogenous catalysts where complex separation steps from the final product are required and more generally contribute to high processing time and costs.
Using low cost reagents which are well known to industry.

The advantage of the present invention compared to prior art, is the development of a new simple and reliable method using a system based on the combination of a Mordenite catalyst with a high boiling point, polar, aprotic glycol ether solvent, for the selective one step conversion of Sclareol to high yield Manoyl oxide; said method can be easily applied in industrial scale as atmospheric pressure and short reaction time is required even in a batch-type reactor. The embodiments of this work comprise solvents which are well known to industry and the low cost, non toxic and environmental friendly mordenite catalyst in order to develop a cost-effective process easily applied in industrial scale. The method presented in this work has an improved selectivity and yield; said method does not lead to the procurement of high quantities of undesirable by-products and selectivity is controlled to produce high yield Manoyl oxide; said method is a process where cyclodehydration of diols to produce cyclic ethers has not the limitation that at least one of the hydroxyl groups of the diol is not a tertiary hydroxyl group.

The method of using a catalytic system which combines high boiling point glyme-type polar aprotic ether solvent and a mordenite catalyst in atmospheric pressure and temperature up to 140°C, for the one-step conversion of Sclareol with high selectivity to Manoyl oxide and yields higher than 85% based on the starting material, has at present never been reported.

Finally, in some embodiments of the present invention, Manoyl oxide is converted to Sclareolide (III). Such chemical reactions do not comprise catalytic reactions. These non-catalytic reactions are well known by prior art. In the current work, Manoyl oxide prepared from claimed method is further oxidized to produce Sclareolide as described in patent US3096346.

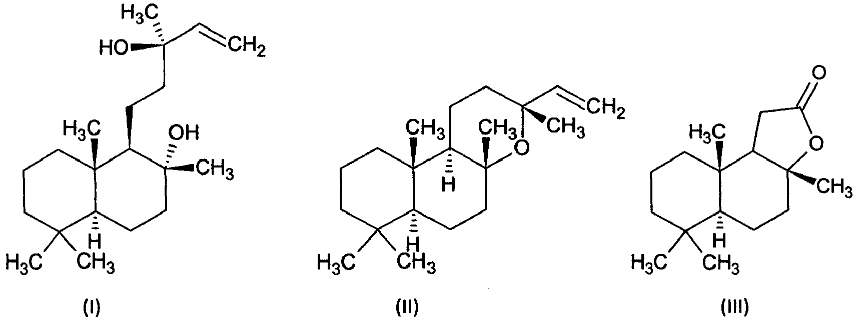

### Summary of the invention

The present invention was made in view of the prior art described above and the it relates to a new method using a system based on the combination of a mordenite catalyst with a high boiling point polar aprotic glycol ether solvent, for the selective one step conversion of Sclareol to high yield Manoyl oxide.

The present invention discloses a method for preparing the diterpene compound Manoyl oxide , including steps of (a) providing a diterpenoid represented by formula (I) (Sclareol), wherein the diterpenoid of formula (I) is extracted from Salvia Sclarea; (b) catalytically dehydrating diterpenoid of formula (I) as a diterpenoid of formula (II)(Manoyl oxide) using a solvent-catalyst system where high selectivity to Manoyl oxide is exhibited and yields higher than 85% based on the starting material are achieved .

More specifically, in the reaction of water elimination in compound (I), the reactivity of oxygen nucleophile of the side chain is successfully controlled to afford a cyclic ether via dehydration, using a system of mordenite catalyst and a high boiling point polar aprotic solvent belonging to the class of glyme-type ethers. This system, the catalyst with the solvent, when it is applied to Sclareol (I) leads to Manoyl oxide (II) with yields exceeding 85% based on the starting material. Atmospheric pressure and temperatures not higher than 140°C are applied. Further oxidation of Manoyl oxide (II) to Sclareolide (III) is performed in a non-catalytic reaction according to previous art.

### Brief description of the drawings

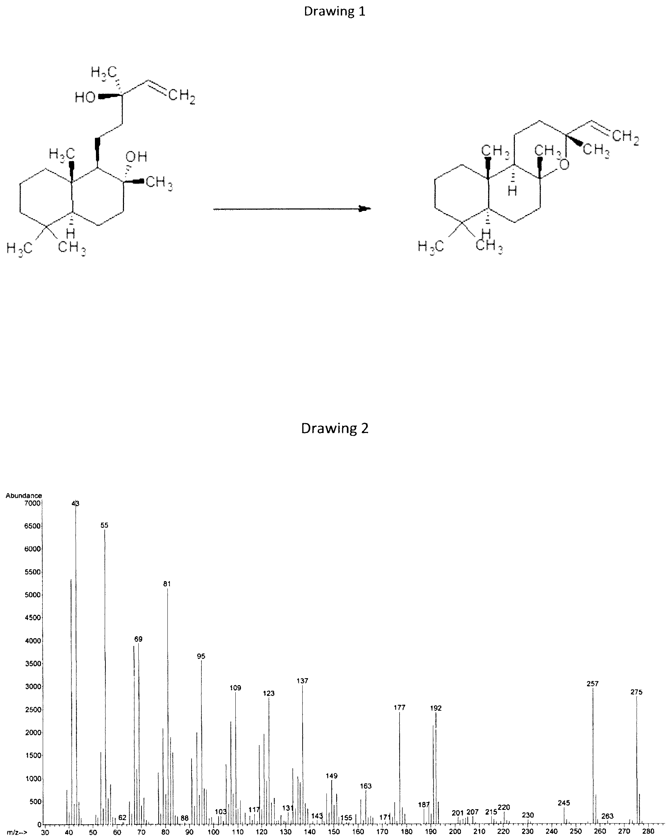

Fig.1 Schematic drawing of the one-step conversion of Sclareol (I) to Manoyl oxide (II) by means described in the present invention
Fig.2 Mass spectrum of Manoyl oxide prepared by means described in the present invention

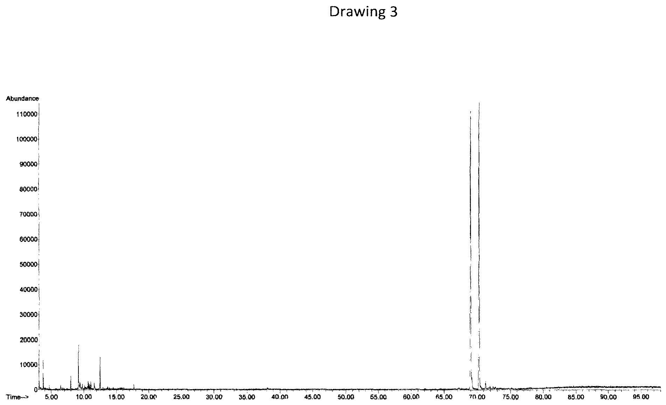

Fig. 3 GC-MS chromatogram of C-13 Manoyl oxide epimers which are prepared by means described in the present invention

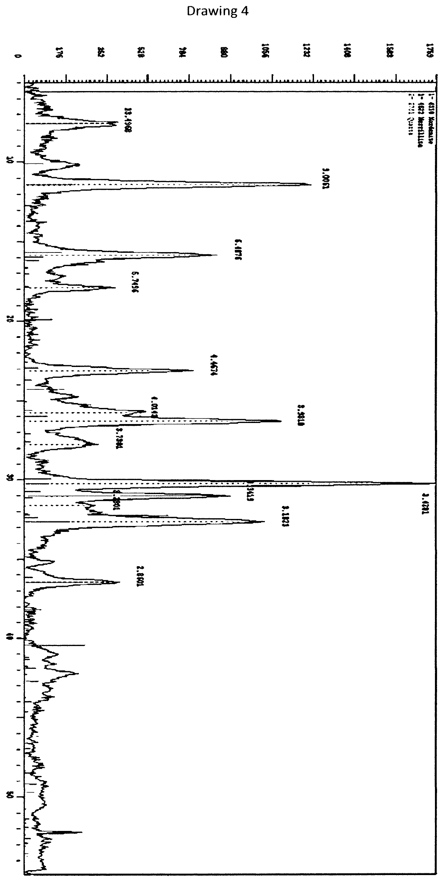

Fig. 4 XRD pattern of a mordenite-based catalyst which is prepared by means described in the present invention and used for the preparation of Manoyl oxide by means described in the present invention

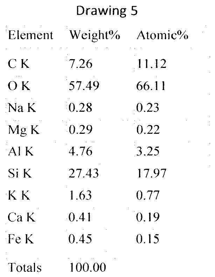

Fig. 5 SEM/EDS analysis of a mordenite-based catalyst which is prepared by means described in the present invention and used for the preparation of Manoyl oxide by means described in the present invention

### Detailed description of the preferred embodiments

Mordenite is one of the most siliceous zeolites. The mordenite used in the present invention is natural and is originated from volcanic soils in Greece. The mordenite catalysts are prepared by means of acid activation of natural mordenite, whereas the Si/Al ratios of the acid-treated mordenites are determined by Scanning Electron Microscopy with Energy Dispersive Spectroscopy SEM/EDS. The present invention can be extended to synthetic mordenites, whereas the synthetic mordenites should have similar properties as those described in the present invention. Preferably, the Si/AI ratio of the mordenites for the present invention may vary from 4 to 10.

Natural mordenite may be preferably acid activated with acids as Sulfuric, or Hydrochloric or Acetic acid. It is preferably acid activated by any means, for example, by immersing the mordenite in an aqueous solution of acid, followed by separation, washing with water, drying and grinding. In the present invention the zeolites used are TECHNOSA-S2, TECHNOSA-S4, TECHNOSA-S6 which are mordenite-type zeolites treated with Sulfuric acid and TECHNOSA-H2, TECHNOSA-A2 which are mordenite-type zeolites treated with Hydrochloric and Acetic acid respectively. All of the above mentioned catalysts are prepared by VIORYL S.A. Natural mordenites which are not subjected to acid treatment, do not exhibit catalytic activity in the reactions studied in the present invention.

Furthermore, the mordenite catalyst may preferably be modified with suitable ions, such as the alkali metals of row 2 of the periodic table; such as the alkaline earth metals of rows 3,4 and 5 of the periodic table; such as the transition metals of rows 4 and 5 of the periodic table and Pt excluding Cd and Tc ; such as the metalloids of row 4 of row periodic table. The ion exchange modification may be carried out by any means, for example, by immersing the mordenite in an ion solution, or by coating or by sedimenting adequate elements onto the mordenite. The minimum of the ion exchange modification in mordenites may be 30% of their ion exchangeable capacity. The elements mentioned above for the ion exchange modification of mordenites may be contained in the mordenite in the form of simple substance atoms, oxides or double oxides.

The modification or alteration methods as described above are not critical. Any acid activated mordenite containing the particular metals or oxides thereof as a part of the components fall within the scope of the invention, as long as they are prepared by acid treatment and then metal exchanging, binding, sedimentation, coating, or any other process. It will be appreciated that various modifications may be made in carrying out the above-described preparation of mordenite catalysts. The mordenite catalysts may be used as they are, or in the forms of tablets, or in any other shaping.

They are used for a conversion reaction of Sclareol (I) or a reaction through dehydration of Sclareol to Manoyl oxide (II), whereas these reactions mentioned above may be carried out in a batch reactor, liquid phase fixed bed or fluidized bed, or in other flowing systems with or without nitrogen gas.

It will be appreciated that various modifications may be made in carrying out synthesis of Manoyl oxide (II) from Sclareol (I). Thus, reaction conditions such as temperatures, solvents, reaction times, can be relatively widely varied without departing from the spirit and scope of the invention. Accordingly, the following procedure is given as representative of only one preferred way of practicing the invention:
Diterpenoid of formula (I) is dissolved in the appropriate solvent, where the appropriate solvent is a high boiling point polar aprotic solvent belonging to the class of glyme-type ethers. The following solvents serve to illustrate the type of solvent described in the process of the invention without limiting the same, therefore the preferred solvent may be glyme type ethers (Dimethoxyethane) such as diglyme (Diethylene glycol dimethyl ether), proglyme (Dipropylene glycol dimethyl ether), Tetraglyme (Tetraethylene glycol dimethyl ether). After the diterpenoid of formula (I) is dissolved, then it is dehydrated over the catalyst at 60-140°C. After 1.5-6 hours, a C-13 epimeric mixture of diterpenoid of formula (II) is obtained in excellent yield, e.g. over 85% based on the starting material. The prepared Manoyl oxide (II) described above, may then be subjected to non-catalytic oxidation, by means described in prior art, to produce Sclareolide (III) in yields up to 75% based on the starting material.

It will be appreciated that the above reaction conditions are only given as representative of a preferred way practicing the invention. The essential feature of the process as illustrated include the new method using a novel catalytic system based on the combination of mordenite catalyst with a high boiling point polar aprotic glycol ether solvent, for the selective one step conversion of Sclareol (I) to high yield Manoyl oxide (II), where the configuration of the substrate is controlled in order to afford a cyclic ether (II) via dehydration by the Catalyst.

The following example will serve to illustrate the process of the invention without limiting the same:

### EXAMPLE: Sclareol-Solvent-Catalyst system for one-step conversion of compound (I) to compound (II)

Catalytic conversion of compound (I) is performed in a batch reactor using Sclareol which is dissolved in a high boiling point polar aprotic solvent belonging to the class of glyme-type ethers. At these tests a predetermined amount of Sclareol and catalyst are added in a round bottom flask in a mass ratio varying from 100:1 to 10:1, more preferably 17:1 and the mixture is heated under reflux and stirred vigorously. The system Sclareol-Solvent-Catalyst is heated to the boiling point of each solvent or to 140°C when solvent with boiling points higher than 140°C are used.

More specifically, the higher yields are achieved when the catalytic conversion of Sclareol is performed at 140°C for a heating period of 1.5 hours and Sclareol is initially dissolved in a glyme type solvent as Diglyme (Diethylene glycol dimethyl ether).At these tests, the yield of Manoyl Oxide epimers (II) is more than 85% based on the starting material, while Sclareol is almost 100% converted as shown in Table 1.

**Table 1**

| | |
|---|---|
| Sclareol conversion | >97% |
| Yields: | |
| C13 Manoyl oxide epimers | >85% |
| Others | <15% |

In case of repeating the above experimental procedure without the addition of the appropriate solvent i.e. Sclareol-Catalyst system, Manoyl oxide (II) yields are up to 50-60% based on the starting material, while Sclareol (I) conversion is higher than 95% .

## Claims

1. A method using a novel catalytic system based on the combination of a mordenite catalyst with a high boiling point, polar, aprotic solvent which belongs to the glycol ethers (Dimethoxyethane), for the one-step conversion of diols with tertiary hydroxyl groups to cyclic ethers in atmospheric pressure, temperatures not higher than 140°C and reaction times equal to 1.5-6 h.

2. The method according to claim 1, wherein the polar, aprotic, high boiling point solvent is Diglyme (Diethylene glycol dimethyl ether), Proglyme (Dipropylene glycol dimethyl ether) and Tetraglyme (Tetraethylene glycol dimethyl ether).

3. The method according to claims 1 and 2, wherein the mordenite is acid treated and has a ratio Si/AI equal to 4 to 10.

4. The method according to claims 1,2 and 3 , wherein this novel catalyst-solvent system is applied for the selective one-step conversion of Sclareol (Labd-14-ene-8,13-diol) to Manoyl oxide (8,13-epoxy-labd-14-ene) in yields higher than 85% based on the starting material.
